# EUROPEAN PATENT SPECIFICATION

(11) **EP 0 912 251 B1**
(45) Date of publication and mention of the grant of the patent: **07.04.2004**
(21) Application number: 97932918.2
(22) Date of filing: 22.07.1997
(51) Int. Cl.: B05B 5/025, D01D 5/00

(54) **A DISPENSING DEVICE AND METHOD FOR FORMING MATERIAL**
VORRICHTUNG ZUM SPENDEN UND VERFAHREN ZUR FORMUNG EINES MATERIALS
DISTRIBUTEUR ET PROCEDE D'OBTENTION DE MATERIAU

(30) Priority: 23.07.1996 GB 9615387; 26.09.1996 GB 9620064
(43) Date of publication of application: 06.05.1999
(60) Divisional application: 03077325.3
(73) Proprietor: BATTELLE MEMORIAL INSTITUTE, Columbus Ohio 43201-2693 (US)
(72) Inventor: COFFEE, Ronald, Alan, Surrey GU27 1HA (GB)
(74) Representative: Beresford, Keith Denis Lewis
(86) International application number: PCT/GB1997/001968
(87) International publication number: WO 1998/003267

(56) References cited:
- EP-A- 0 005 035
- EP-A- 0 234 841
- EP-A- 0 234 842
- EP-A- 0 250 102
- EP-A- 0 250 164
- WO-A-94/13266
- WO-A-95/26235
- US-A- 4 043 331
- US-A- 4 565 736
- US-A- 4 657 793
- DATABASE WPI Week 9602, Derwent Publications Ltd., London, GB; AN 96-018586, XP002046662 & RU 2 034 534 C (EKOMEDSERVIS) 05 October 1995
- DATABASE WPI Week 9544, Derwent Publications Ltd., London, GB; AN 95-342809, XP002046663 & RU 2 031 661 C (EKOMEDSERVIS) 27 March 1995
- PATENT ABSTRACTS OF JAPAN vol. 015, no. 392 (C-0873), 4 October 1991 & JP 03 161502 A (I C I JAPAN KK), 11 July 1991,

## Description

This invention relates to methods and devices for forming material. In one example, this invention relates to methods and devices for applying material to a surface, for example to an internal or external surface of an animal, for example for applying material to skin for use, for example, in the care or treatment of wounds or burns.

Various forms of aerosol devices for allowing material to be sprayed onto a surface such as the human skin are known, including aerosol devices for spraying wound care products onto wounds or burns. One such product is Savlon Dry (trade mark) which has been marketed in the UK by Zyma Healthcare and Ciba Geigy plc. Such products require the use of a gas propellant and in recent years the choice of gas propellants has become more limited because of the desire to avoid environmentally unfriendly compounds such as a chlorofluorocarbons or hydrocarbons. Also because small droplets and powder particles tend to be carried away from the target by the gas flow created when the propellant gas hits and is deflected by the target surface, such gas propelled sprays are generally designed to spray relatively large droplets or powder particles in order to achieve sufficient inertia to deposit the spray on its target surface. Such gas propelled products may run if sprayed too freely, especially where the spray produces large droplets. In addition, the packaging costs for such devices are high.

GB-A-1569707 describes a dispensing device for producing a spray or cloud of liquid droplets intended primarily for crop spraying. The process described in GB-A-1569707 produces liquid droplets by applying an electric field to a liquid emerging from an outlet in the vicinity of the surface so that the liquid becomes sufficiently charged that the net electric charge in the liquid as the liquid emerges into free space counteracts the surface tension forces of the liquid and the repulsive forces generated by the like electrical charges cause the liquid to be comminuted to produce a cone or jet which breaks into liquid droplets. The droplets produced by this device are charged close to their Rayleigh Limit and thus in use migrate quickly toward conductive surfaces of lower or zero potential. This technique of comminuting liquid is generally known as electrohydrodynamic comminution or processing.

RU-A-2 034 534 discloses a method and device for electrohydrodynamically comminuting and applying to the skin a protective cooling consisting of an aseptic solution containing a fibre forming polymer.

In one aspect, the present invention provides a method of forming composite matter, which comprises supplying a liquid to a first outlet and supplying a further liquid to a second outlet located adjacent the first outlet and subjecting the liquids to an electric field so as to form at least one jet, the liquids being such that, after formation, the jet forms composite matter comprising at least one of a fibre, fibre fragments and particles, the composite matter having a core formed substantially by one of the two liquids and a coating formed substantially by the other of the two liquids.

In another aspect, the present invention provides a device comprising a housing (9) containing:
a first reservoir (20a) containing a first liquid;
a second reservoir (20b) containing a second liquid;
first liquid supplying means for supplying the first liquid to a first liquid supply outlet;
second supplying means for supplying the second liquid to a second liquid outlet adjacent to the first liquid outlet; and
means for subjecting liquid issuing from the first and second liquid outlets to an electric field to cause the liquids to form at least one jet of electrically charged liquid, the liquids being such that, in use of the device after the at least one jet has been formed, the jet forms composite matter comprising at least one of a fibre, fibre fragments and particles, the composite matter having a core formed substantially by one of the first and second liquids and a coating formed substantially by the other of the first and second liquids.

Where the resulting matter or material is to be applied or supplied to a cavity or concave surface, then desirably the matter is at least partially electrically discharged before application or supply.

In an embodiment, the deposition process may be repeated one or more times to provide a number of layers of material comprising at least one of fibres, fibrils, droplets or particles on the surface. The polarity to which the material is charged may be reversed between deposition of different layers so as facilitate attraction between the layers.

The liquid used to produce the electrohydrodynamically formed matter may comprise a biologically active ingredient or component. Where the electrohydrodynamically formed material comprises fibrils, the fibrils may actually stick into the skin of soft tissue enabling delivery of the active component to a location beneath the outer layer of skin or soft tissue.

The liquid(s) used may comprise a solution, suspension, microsuspension, emulsion, microemulsion, gel or even a melt which may contain an active component or components. Microcapsules, fibres or fibrils of a bioresorbable or biodegradable polymer may be formed which contain a biologically active ingredient. Material from the core of fibre or fibril may be released from the ends of the fibre or fibril. Material from the core of a fibre, fibril or microcapsule may be released through the coating if the coating is permeable to the material contained within it or may be released as a result of the outer coating being breached, for example by chemical or enzymic attack which causes the outer coating to dissolve or degrade, by bioresorption or biodegradation of the coating, or as a result of temperature changes or application of pressure which causes the outer coating to rupture. The timing of the release may be controlled, for a given polymer, by controlling the thickness of the coating surrounding the core.

Possible biologically active components for topical application are pharmaceutical compounds such as analgesics, antiseptics, antibiotics, antifungals, antibacterials, antiparasitics, debridement agents such as proteolytic enzymes, biological products such as cells, and cytokines for stimulating cytokinetic activity to promote essential cell activities, for example, to stimulate dendritic growth, growth factors such as fibroblast growth factor (FGF), epithelial growth factor (EGF), transforming growth factor (TGF) and others that may be used to promote or otherwise control the sequence of events essential to natural tissue repair, DNA or other genetic material for gene therapy, cells, peptides or polypeptides, insulin, adjuvants, immune suppressants or stimulants, surface binding or surface recognising agents such as surface protein A, and surfactants. Where more than one layer of fibres, fibrils or droplets is deposited, then different active ingredients may be provided in different layers.

Fibres, fibre fragments or particles of biological material such as fibrin or collagen may be formed using a method embodying the invention. Also electret polymers may be used to act as nuclei or otherwise initiate interactive cellular and/or molecular events in tissue repair.

A number of electrohydrodynamic processing sites may be provided enabling different types of electrohydrodynamically formed matter to be deposited at the same time.

The deposited material may be used alone or in combination with a conventional bandage or dressing. As another possibility, where the material contains, for example, a therapeutic agent, the material may be deposited onto a conventional dressing to be applied to the skin.

The fibrils or particles may comprise biologically active material, for example the fibrils or particles may comprise DNA encapsulated in or complexed with a lipid for transfecting cells or may, for example, contain or encapsulate matter such as peptides, polypeptides and other large biomolecules such as insulin or growth factor, and/or active pharmaceutical components for enabling delivery of the active component into the blood stream via the lung. This should provide a quicker route to the bloodstream than that provided by normal oral ingestion and avoids the need for injection of components which cannot be taken orally because of the gastric enzymes and acids present in the digestive system.

Microcapsules or fibrils for oral ingestion of appropriate active components enabling slow release of those components may also be produced by electrohydrodynamic means by providing the active component as the core of the capsule or fibril.

Embodiments of the present invention will now be described, by way of example, with reference to the accompanying drawings, in which:
Figure 1 shows schematically one example of a device for carrying out electrohydrodynamic processing;
Figures 2a to 2c are schematic diagrams for illustrating the mechanisms by which at least partially solid or gel-like particles, fibrils and fibres, respectively, may be produced;
Figure 3 shows schematically another example of a device for carrying out electrohydrodynamic processing;
Figure 4 shows schematically use of the device shown in Figure 3 to apply a dressing to the skin surface, a wound, burn or area exposed by a surgical procedure.
Figures 5 to 8 illustrate various different types of nozzles or outlets which may be used in an electrohydrodynamic processing device;
Figure 9 shows a mat or web of fibres produced by electrohydrodynamic processing;
Figure 10 shows substantially parallel fibres deposited on a surface by electrohydrodynamic processing;
Figure 11 shows a part cross-sectional view of an example of a device for use in a method embodying the invention;
Figures 12 shows a nozzle which may be used to produce composite material;
Figure 13 shows a nozzle for producing material from a mixture of two different liquids; and
Figure 14 shows schematically another example of a device for carrying out a method embodying the invention.

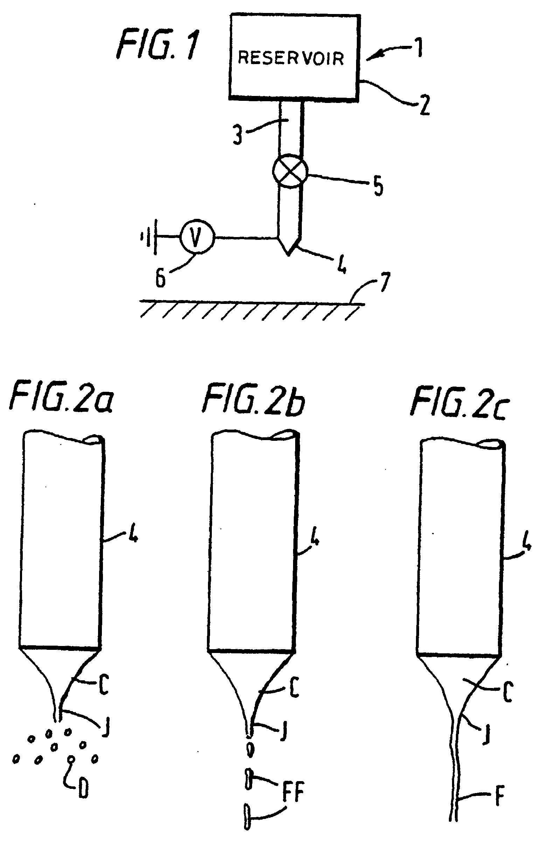

Referring now to the drawings, Figure 1 shows schematically a device 1 for carrying out electrohydrodynamic processing. The device 1 comprises a container or reservoir 2 of liquid coupled by a supply pipe 3 to an outlet 4 via a flow regulating valve 5 of conventional form. The valve 5 may be a manually or electrically operable valve. A voltage source 6 supplying a voltage of typically 15 to 25kV is coupled to the outlet 4 so as to cause liquid issuing from the outlet 4 to become charged. If the liquid is at least semiconducting (that is the liquid has a resistivity below about 10⁹ ohm-m), the voltage source 5 may be coupled to the liquid upstream of the outlet 4.

In use of the device 1, a surface area 7 such as an area of the skin of an animal, for example an area of skin of a human being, is positioned a few centimetres, for example from 5 to 10 cm, below the outlet 4 as shown schematically in Figure 1. The voltage source 6 is coupled to the outlet 4 by closing a switch (not shown in Figure 1) and the flow regulating valve 5 opened so that liquid is supplied under gravity to the outlet 4. The liquid is selected to be biologically compatible, that is not harmful or detrimental to the animal when deposited on its skin or an open wound, and will typically have a resistivity in the range of from approximately 10² to 10⁸ ohm-metres and a viscosity in the region of from 0.1 to 1000 Poise or greater with the viscosity being dependent on whether a fibre, fibre fragments or segments or particles are to be formed.

As described in the aforementioned GB-A-1569707 and an article entitled "Electrodynamic Crop Spraying" by R. A. Coffee published in Outlook on Agriculture Volume 10 No. 7 1981, liquid issuing from the outlet 4 is subject to an intense electrical field which establishes a standing wave along the surface of the liquid producing cusps or cones which emit jets of charged liquid.

The small perturbations which inevitably occur in the liquid jet cause the jet to become unstable and the net electrical charge in the liquid provides a repulsive force which acts against the surface tension forces in the liquid. This would normally be expected, as described in GB-A-1569707, to cause the liquid to break up into droplets which, because both they and the outlet 4 are similarly charged, are propelled away from the outlet 4 and each other so providing a spray or cloud of liquid droplets. The present inventors have, however, found that by selecting the liquid and controlling the conditions of the electrohydrodynamic process, the jet of liquid, rather than breaking up into liquid droplets, forms a solid or gel-like fibre or forms fibre fragments (fibrils) or non-liquid droplets or particles. In use of the apparatus shown in Figure 1, the fact that the electrohydrodynamically produced material is charged and the animal body can effectively be considered earthed causes the material to deposit onto the surface 7 of the skin beneath the outlet 4. The material deposits swiftly, uniformly and gently by the energy contained in the electric field used to generate the material and will not overspray, nor become trapped in air streams and swept away from the target surface. One or more layers of such material may be deposited to provide a dressing to, for example, cover or protect, a wound or burn. This material being non-liquid should not cause the irritation which may arise from, for example, solvents if liquid droplets were applied to the skin.

Relative movement may be effected between the nozzle 4 and the surface 7, in this example the surface 7 may be moved, to enable coverage of a large area.

Figure 2a illustrates the situation where the liquid supplied to the outlet or nozzle 4 forms a spray of solid droplets or particles D while Figure 2b illustrates the situation where the liquid jet breaks up into fibrils FF and Figure 2c illustrates the situation where the liquid jet J forms a fibre F.

Figures 2a to 2c show only one cone C and the associated jet J emanating from a nozzle 4. The actual number of cone and jets produced will, however, depend upon several factors, including the resistivity, permittivity and flow rate of the liquid, the dimensions of the outlet 4 and the applied electric field.

In order to form the solid or gel-like droplets shown in Figure 2a, the liquid is selected or formulated so as to become non-liquid, that is at least partially solid or gel-like, after the liquid has been separated by the applied electric field into liquid droplets. Where the liquid includes a solvent, this may be achieved by, for example, selecting a liquid of such a volatility and viscosity and controlling the flow rate so that the solvent evaporates sufficiently to cause at least partial solidification or gellification only after droplet formation. Where the liquid is a melt which is held at an elevated temperature during supply to the outlet, then the liquid should be selected to have a melt temperature such that the liquid solidifies after liquid droplet formation. This may be facilitated by quenching using, for example, a cold inert gas or air stream.

To form the fibrils or fibre fragments shown in Figure 2b, the liquid is selected or formulated and the flow rate controlled so that the liquid jet becomes at least partially non-liquid, that is solid or gel-like, before the liquid has been separated by the applied electric field into liquid droplets but so that the growth wave resulting from perturbation of the jet J remains sufficiently strong to inhibit formation of a fibre and causes the jet to break up into fibre fragments or fibrils FF. This may be achieved by selecting the liquid and flow rate so that the liquid begins to solidify (for example by evaporation in the case of a solution or by cooling in the case of a melt) before droplet formation and becomes relatively brittle so that the growth wave causes the nascent fibre to break into segments. Break up of the nascent fibre into fibrils may be facilitated by pulsing the voltage applied to the outlet 4 so as the create an energy pulse which sets up a resonant process to promote breaking up of the nascent fibre. Experiments have shown that the length of the fibrils is related to the pulse duration or frequency with, under ideal conditions, the fibril length being equal to the jet velocity divided by the pulse frequency so that, for example, if the jet velocity is 5ms⁻¹ and a pulse frequency is 100kHz is used, the fibrils should have a length of 50µm. Fibrils having lengths in the region of, for example, tens of micrometres to a few centimetres may be produced, depending upon the particular liquid and electrohydrodynamic process conditions used.

In order to form the solid or gel-like fibre F shown in Figure 2c, the liquid is selected so as to become non-liquid, that is at least partially solid or gel-like, after issuing from the outlet, and the growth wave resulting from perturbation of the jet is attenuated so that the jet does not break up but forms a continuous fibre which has a length determined by the time for which the electrohydrodynamic process is continued, that is the time for which the voltage is applied. Attenuation of the growth wave may be achieved by the incipient solidification and/or by the nature of the liquid. Fibre production may be achieved by, for example, selecting a liquid which is highly volatile or has a highly volatile component so that solidification by evaporation occurs very quickly before droplet formation. For example fibres may be formed using a liquid comprising a polymer which on solidification tends, because of its viscosity and/or polymer chain morphology, to resist growth wave development. Fibres may be formed using a relatively high molecular weight polymer, for example a polymer having a molecular weight in the region of 140000 or more. Where the liquid used is a melt then choosing a liquid which solidifies to a relativity plastic state should promote fibre formation.

The device 1 shown in Figure 1 uses a gravity feed to supply liquids to the outlet 4 which has the advantage of simplicity. It is most suitable for use in situations where the area of skin to which the dressing is to be applied can easily be moved beneath the outlet 4 or for use when the liquid to be supplied may be detrimentally affected by pumping.

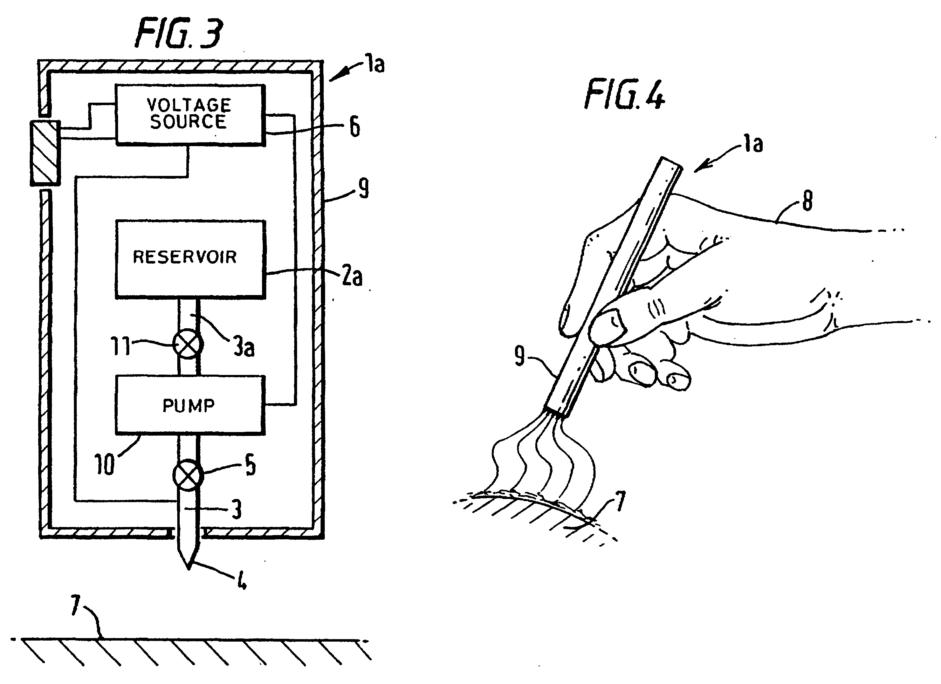

Figure 3 illustrates a part cross-sectional view of another form of electrohydrodynamic processing device 1a. The device shown in Figure 3 is, as illustrated schematically in Figure 4, intended to be portable, in particular so as to be held in the hand 8 of a user.

The device 1a shown in Figure 3 comprises a housing 9 within which is mounted a reservoir 2a of the liquid to be dispensed. The reservoir 2a may be formed as a collapsible bag so as to avoid any air contact with the liquid being dispensed. The reservoir 2a is coupled via a supply pipe 3a to a pump chamber 10 which is itself coupled via the supply pipe 3 and the flow regulating valve 5 to the outlet 4 in a similar manner to that shown in Figure 1. The voltage source 6 in this example is coupled to a user-operable switch SW1 which may be a conventional push button or toggle switch, for example. The voltage source 6 may comprise, for example a piezoelectric high voltage source of the type described in WO94/12285 or a battery operated electromagnetic high voltage multiplier such as that manufactured by Brandenburg, ASTEC Europe of Stourbridge West Midlands, UK or Start Spellman of Pulborough, West Sussex, UK and typically provides a voltage in the range of from 10 to 25kV. Although not shown, a voltage control circuit comprising one or more resistor capacitor networks may be provided to ramp the voltage up smoothly. The reservoir 2a may be coupled to the pump chamber 10 by way of a valve 11 which may be a simple non-return or one way valve or may be an electrically or mechanically operable valve of any suitable type, for example a solenoid or piezoelectric valve, operable by a voltage supplied by the aforementioned control circuit.

The pump chamber 10 may comprise any suitable form of pump, which provides a continuous substantially constant flow rate, for example an electrically operable pump such as a piezoelectric, or diaphragm pump or an electrohydrodynamic pump as described in EP-A-0029301 or EP-A-0102713 or an electroosmotic pump as described in WO94/12285 or a mechanically operable pump such as syringe pump operated or primed by a spring biassing arrangement operable by a user.

In use of the device 1a shown in Figures 3 and 4, the user first positions the apparatus over the area 7 to which the material is to be applied, then actuates the switch SW1 and the pump of the pump chamber 10 to cause, when the valves 5 and 11 are opened, a stream of liquid to be supplied to the outlet 4 whence the liquid is subjected to the applied electric field as described above with reference to Figures 2a to 2c, forming charged matter which deposits onto the said surface 7 which may be the skin or on or within a wound. The user may move the device 1a relative to the area 7 to cover a large area. One or more layers may be formed in a manner similar to that described with reference to Figure 1. The device shown in Figures 3 and 4 has, however, the advantage that it is portable so allowing it to be used for, for example, first aid at the site of an accident and/or on relatively inaccessible areas of the body and does not rely on gravity feed.

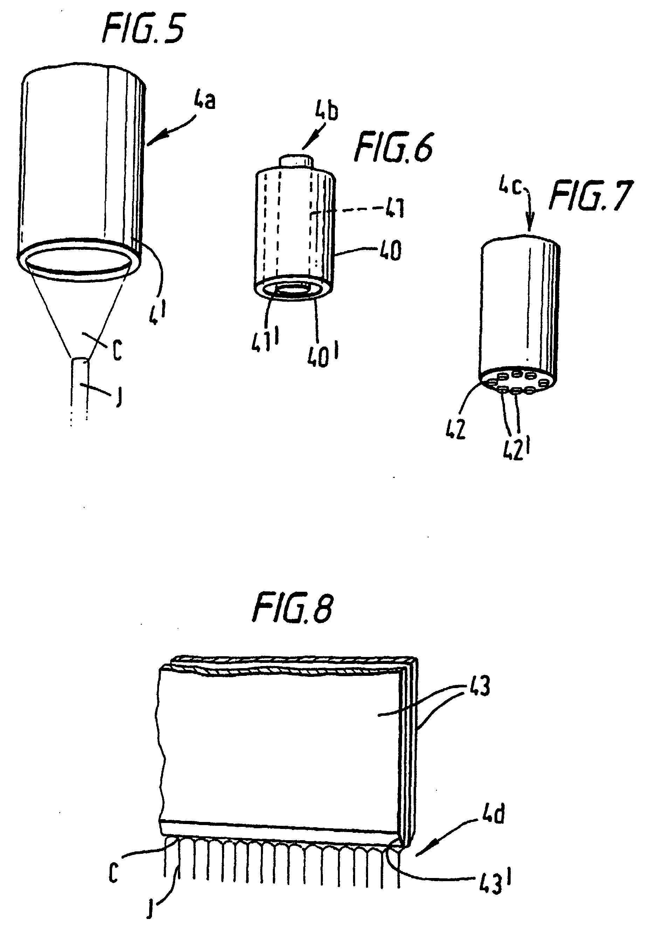

Various different forms of outlet or nozzle 4 may be used in the devices shown in Figures 1 and 3 and 4. Figures 5 to 8 illustrate schematically some examples. Another possibility is the fibre comminution site or nozzle described in WO95/26234.

The nozzle 4a shown in Figure 5 comprises a hollow cylinder which is conductive or semiconductive material at least adjacent its end 4' where the voltage is to be applied in use and will in use produce one or more jets (one cusp or cone C and jet J are shown) depending upon the resistivity and flow rate of the liquid and the voltage applied to the outlet 4.

The nozzle 4b shown in Figure 6 comprises two coaxial cylinders 40 and 41 at least one of which is conductive or semiconductive at least adjacent its end 40' or 41' where the voltage is applied and will in use produce a number of jets depending upon the resistivity and flow rate of the liquid and the applied voltage.

The nozzle 4c shown in Figure 7 comprises a number of parallel capillary outlets 42 which are conductive or semiconductive at least adjacent their ends 42' where the voltage is applied. Each capillary outlet 42 will normally produce a single jet. The multiple nozzles shown in Figure 7 have the advantage that blockage of one nozzle by relatively viscous liquid does not significantly affect the operation of the device and also allow different liquids to be supplied from respective reservoirs to different ones of the nozzles.

The nozzle 4d shown in Figure 8 comprises a slot-shaped nozzle defined between two parallel plates 43 which are conductive or semiconductive at least adjacent their ends 43' where the voltage is applied. The use of a slot nozzle when relatively highly viscous liquids are being used is advantageous because complete blockage of the nozzle is unlikely, as compared to the case where a relatively fine capillary nozzle is used, and a partial blockage should not significantly affect the functioning of the device because the liquid should be able to flow round any such partial blockage. The use of a slot-shaped nozzle outlet as shown in Figure 8 also allows a linear array of jets and thus of fibres, fibrils or particles or non-liquid droplets to be formed.

Where, as discussed above, the liquid being used is sufficiently conductive to enable the voltage to be applied to the liquid rather than the nozzle then the nozzle may be formed of any suitable electrically insulative material which does not retain electrical charge for any significant length of time, for example glass or a semi-insulating plastic such as polyacetyl.

The nozzle shown in Figure 7 is designed to produce a single jet per individual outlet 42. The nozzles shown in Figures 6 and 8 will in use produce a number of jets which extend generally along the electric field lines, with the number of jets depending upon, of course, the length of the slot (Figure 8) or the diameter of the annulus (Figure 6) and also upon the resistivity of the liquid, the flow rate and the applied voltage.

In the case of the cylindrical nozzle shown in Figure 5, when the flow rate is high only one jet will be produced as shown. However, at low flow rates, the liquid tends to emerge from the outlet as a film which clings to the rim of the cylinder and there forms multiple jets in a manner analogous to the annular nozzle shown in Figure 6.

Where the resistivity of the liquid is high, for example about 10⁹ ohm-m, some 10 or 20 jets, dependent upon the applied voltage and flow rate, may be formed per cm length of the nozzle, allowing the same number of fibres, for example, to be produced (spun). The applied voltage also affects the diameter of the resulting material. Thus, about 10 to 15 fibres of about 10 to 20 micrometres in diameter may be formed per cm length of the slot shown in Figure 8 from a liquid having a resistivity of about 10⁹ ohm-m when the applied voltage is 15 kilovolts and a larger number, about 20, of fibres of smaller diameter may be formed per cm length of the slot when the applied voltage is 25 kilovolts. At liquid resistivities of, for example, 10⁷ ohm-m, some 5 to 10 fibres may be spun per cm length of the slot, dependent again on the applied voltage and flow rate, with again a larger number of thinner fibres being formed at higher voltages. The number of jets produced decreases but their diameter increases with increasing flow rate. By selecting the resistivity and viscosity of the liquid, the flow rate and the applied voltage, material, for example fibres or fibrils, with diameters from a few, about 10 nanometres (nm) to above 100 micrometres, typically 10² to 10⁴ nm, may be produced. Similar results may be achieved using the hollow cylinder nozzle of Figure 5 or the annular nozzle of Figure 6.

The use of a liquid which is controlled to produce fibres is particularly advantageous for producing a wound or burn dressing because, as will be described below, deposition of the fibres onto the area being covered results in a network of crossing or interlinking fibres providing effectively an integral web or mat which has a high specific surface area and is thus highly absorbent to fluids, whilst being exceptionally light. Like a conventional dressing it enables good coverage over an area of skin so as, for example, to protect a wound but, unlike many conventional dressings, still enables, by virtue of the gaps between the network of fibres, air to pass through the dressing to the wound and pus and other detritus to pass from the wound, while preventing ingress of bacterial matter into the wound.

By controlling the diameters of the fibres in the manner described above and/or by controlling the number of layers of fibres, dressings having a range of thickness, fluid permeability and mechanical strength can be formed enabling the dressing to be adapted for use on different types of wounds and burns including wounds arising from severe trauma such as say motor vehicle accidents, battle wounds etc, and chronic wounds including lesions such as ulcerated veins as well as, where appropriate, surgically exposed tissue. The permeability of the dressing has been found to be a function of the diameters and spacing of the fibres and the motion of the nozzle over the deposition area during application.

Liquids which form short fibrils or solid droplets will not generally form a cohesive mat or web of fibres. However, liquids which form fibrils or solid droplets may be used in combination with conventional dressings or with dressings formed by fibres as discussed above, for example fibrils or solid droplets produced using a method embodying the invention may be deposited into or on a wound and then covered with one or more layers of fibres produced by method embodying the invention or by a conventional dressing.

Fibres, fibrils or droplets produced by electrohydrodynamic processing may be deposited onto a substrate, such as a dressing, for later application to the skin, a wound, burn or the like.

Experiments have been carried out with a number of different polymers and solvents. It has been found that long chain heavy molecular structures facilitate fibre production while short chain length molecular structures tend to form fragments or solid droplets. Solvents which evaporate quickly during the jet flow may be used to facilitate formation of fibres. Suitable solvents may be, for example, methanol, propanol and water, methylene chloride, acetone and chloroform, depending upon the particular polymer used.

Experiments have been carried out in which the device shown in Figure 1 was used with water and hydrocarbon based solutions supplied to a slot-like nozzle of the type shown in Figure 8 having a slot width of about 150 micrometres and a slot length of 2cm. Liquid flow rates of from 1 to 10 microlitres per second and voltages of from 10kV to 15kV were found to produce about 5 to 15 charged fibres per cm length of the slot with the fibres having diameters in the range of from 1 to 100 micrometres.

Fibres have been successfully spun with polyhydroxybutyric acid, a bioresorbable polymer, and polyvinyl alcohol (PVA), a polymer soluble in water and alcohols such as methanol or propanol, and pharmaceutical preparations for wound care, such as "New Skin" (trade mark) marketed by SmithKline Beecham which comprises nitrocellulose in an organic solution (in particular it comprises ethyl acetate, isopropyl alcohol, amyl acetate, isobutyl alcohol, denatured alcohol, camphor and nitrocellulose). "New Skin" is normally applied to scratches and light wounds with a rod or paddle because it is too viscous to be applicable by conventional spray devices. "New Skin" has however been successfully sprayed by a method embodying the invention to form fibres of approximately 0.5 to 5 micrometres diameter which deposited uniformly onto skin, resulting in a firm skin-like web-film. In one specific example neat (that is undiluted) "New Skin" was supplied at a flow rate of 4 millilitres per hour to a capillary nozzle of the type shown in Figure 5 in the form of a 1.1mm diameter thin-walled metal, generally stainless steel, tube. A voltage of 8.2kV was applied to the nozzle which was located approximately 50mm above an earthed deposition surface. Multiple fibres were formed and substantially uniformly deposited on the surface. Fibres have also been produced using undiluted "New Skin" (trade mark) with flow rates of from 1 millilitres per hour to 100 millilitres per hour.

Polyvinyl alcohol (PVA) has also been deposited in a similar manner to the "New Skin", using combinations of alcohol and water as solvent. Neat, undiluted PVA having a molecular weight of typically 15000 has been found to tend to form solid droplets when electrohydrodynamically processed while PVA having a molecular weight of about 140000 or more tends to form fibres. Low molecular weight PVA in a volatile solvent such as ethanol tends to break up into fibrils rather than continuous fibres. Thus, PVA having a molecular weight in the region of about 90000 to 140000 will tend to form fibrils and PVA fibrils having diameters of a few hundred nanometres and lengths of 0.5 to 10mm have been produced.

In another experiment, an annular nozzle of the type shown in Figure 6 was used to which a voltage of from 5kV to 15kV was applied. A 90% by volume solution of poly β-hydroxybutric acid (which is a bioresorbable polymer) in methylene chloride was supplied at a flow rate of from 5 micro litres per second to 50 micro litres per second to the nozzle which was located at a distance of about 5cm from human skin. A covering layer of fibres was formed on the skin with the fibres having diameters, dependent on the applied voltage and flow rate, in the range of from about 10 micrometres to about 50 micrometres.

In another example, the apparatus shown in Figure 1 was used with a thin-walled, generally stainless steel, capillary nozzle of the type shown in Figure 5 having a 1.1mm external diameter. The reservoir was filled with polylactic acid having a molecular weight of 144000 dissolved 10% by mass in acetone and the flow regulator was controlled to provide a flow rate of 10 millilitres per hour. A voltage of 12 kV was applied to the nozzle which was located 8cm away from and perpendicular to a flat earthed counter electrode provided to simulate a skin surface. This experiment was also repeated using a flow rate of 6.0 millilitres per hour and a nozzle voltage of 11.4kV. The surface of the flat plate was covered by a network or mass of randomly distributed fibres having diameters typically in the region of from 2 micrometres to 7 micrometres.

The fibres deposit readily onto capacitive or earthed surfaces without any of the normal problems of applying very low mass high specific surface materials and the electrical field ensures that the fibres deposit swiftly, gently and substantially uniformly.

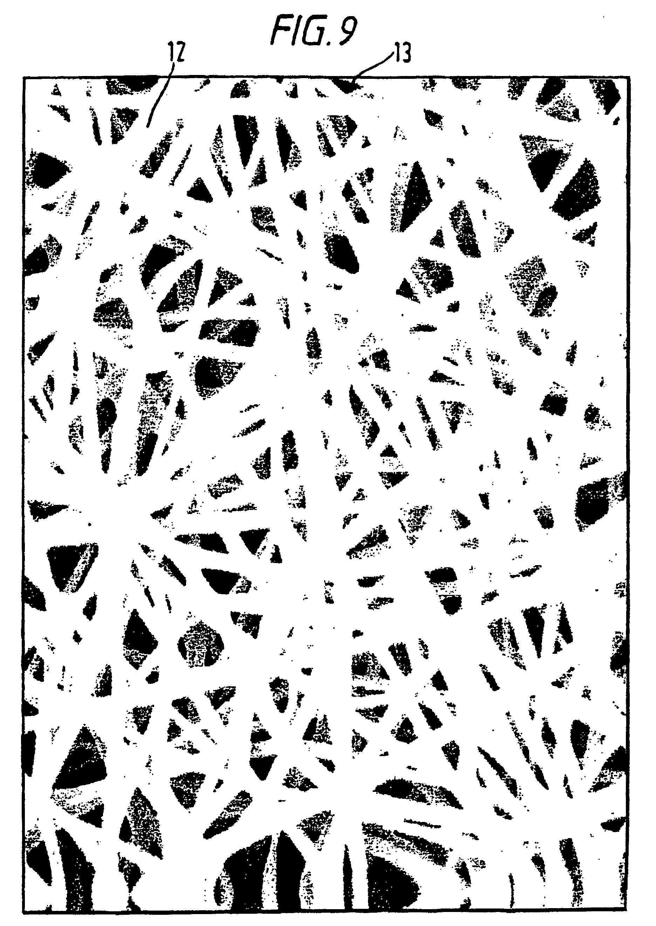

Figure 9 shows a copy of an image produced by scanning electron microscope of a typical mat or web of fibres 12 on a plate 13. The fibres have, typically, a diameter of approximately 5µm. The fibres shown in Figure 9 are relatively randomly distributed because their relatively low mass, and thus low inertia, and high charge to mass ratio means that their movement and thus location of deposition on the surface is strongly influenced by the fact that they are all similarly charged fibres. This also results in the fibres crossing one another and possibly even blending together which should increase the overall mechanical integrity of the web or mat.

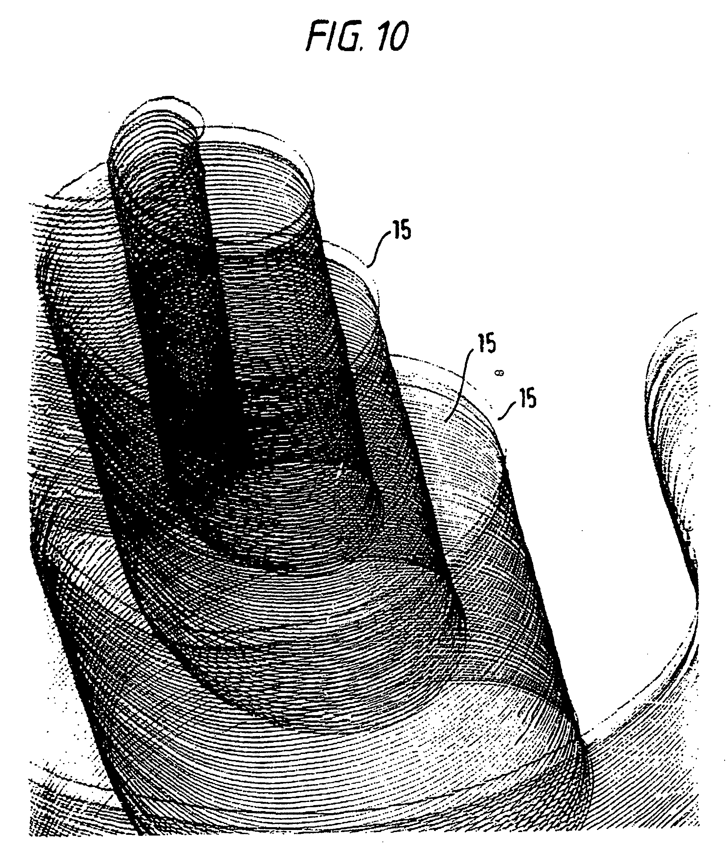

By increasing the mass of the fibres and thus their inertia, and reducing their charge to mass ratio, greater control can be achieved over the deposition of the fibres so that the location at which the fibres are deposited on the skin or wound can be controlled mainly by moving the nozzle relative to the skin or wound and by controlling the number of passes and pattern of movement of the nozzle over the surface. Figure 10 shows an example of fibres 15 of about 50 to 100 micrometres in diameter deposited onto a substrate using a slot-shaped nozzle of the type shown in Figure 8. As can be seen from Figure 10, a single pass of the nozzle produces a set of approximately parallel tracks and, with two or more passes, a relatively dense material akin to a textile can be produced. Although the actual pattern shown in Figure 10 was produced by depositing fibres of a heavy build viscous paint onto paper, it will be appreciated that similar results can be achieved using other material such as inert polymers of similar mass. The movement of the nozzle may be controlled to produce any desired pattern and that for example a woven texture could be simulated. Such fibres may be used, for example to form a bandage.

In the examples described above, the fibres, fibrils or droplets produced using the method embodying the invention consist simply of an inert polymer which may be a bioresorbable polymer such as polyhydroxybutyric acid, polyvinyl alcohol, polyglycolic acid or polylactic acid. Biologically active ingredients may, however, be added to the liquid before it is supplied to the outlet nozzle 4. In such cases, the liquid may comprise a solution, suspension, microsuspension, emulsion, microemulsion, gel or even a melt containing the active component or components. Possible active components are one or more of the following, namely pharmaceutical compounds such as analgesics, antiseptics, antibiotics, bactericides, antifungals, antiparasitics, anti-inflammatory agents, vasodilators (such as minoxidil which is believed to promote wound epithelialization and neovascularization), agents such as proteolytic enzymes for debridement and tissue repair promoting materials such as for example cytokines for stimulating cytokinetic activity to promote essential cell activities, for example to stimulate dendritic growth, growth factors such as fibroblast growth factor (FGF), epithelial growth factor (EGF), transforming growth factor (TGF) that are believed to reduce scarring and others that may be used to promote or otherwise control the sequence of events essential to natural tissue repair, cells, peptides, polypeptides, insulin, immune suppressants or stimulants and vaccines. Another possible active components are DNA or other genetic matter for gene therapy, surface binding or surface recognising agents such as surface protein A, and surfactants.

Where more than one layer of fibres, fibrils and/or particles is deposited, then different active ingredients may be provided in the different layers and different biologically active ingredients may be included in different fibres, fibrils or particles where a nozzle of the type shown in Figure 7 is used. Also biologically active ingredients may be provided between layers, for example skin cells may be interspersed in or between layers.

The active ingredient may comprise an adjuvant that is a pharmacological agent added to a drug to increase or aid its effect or an immunological agent that increases the antigenic response.

Where the resulting material is in a form of fibrils, the fibrils may actually stick into the surface, for example skin or soft tissue, onto which they are deposited so enabling, for example, the supply of drugs and other biologically active agents beneath the skin or into the soft tissue, and may for example be used to carry DNA to cells.

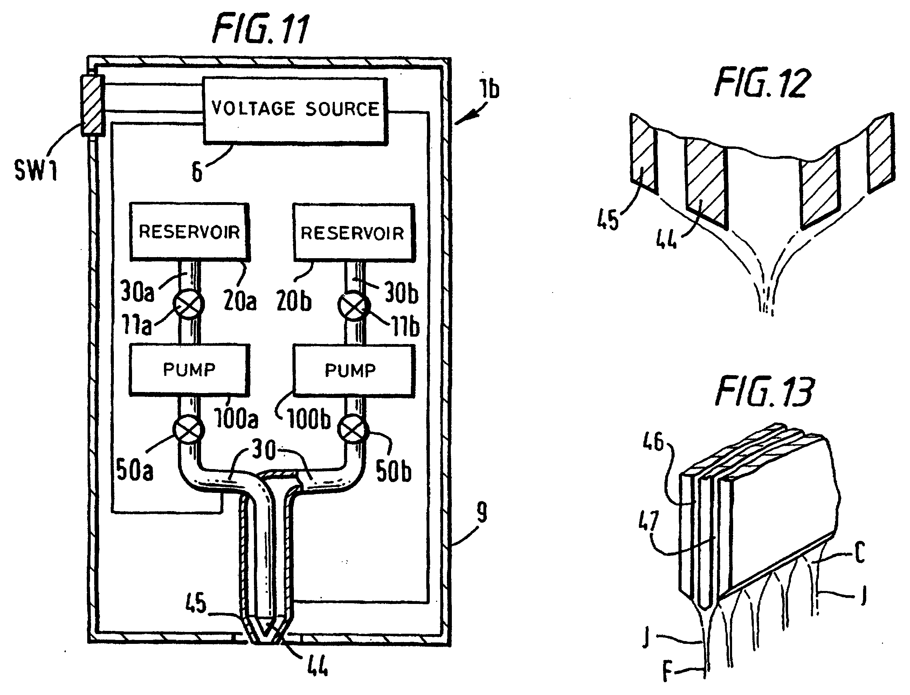

Figure 11 illustrates a device 1b embodying the present invention. The device 1b shown in Figure 11 is similar to that shown in Figure 3 but comprises two reservoirs 20a and 20b each coupled by respective supply pipes 30a and 30b and possibly by non-return valves 11a and 11b to a respective pump chamber 100a and 100b coupled via a respective valve 50a and 50b to a respective liquid supply pipe 30 which terminates in a respective outlet 44 and 45 arranged so that the outlet 45 is coaxial with and extends around the outlet 44. Figure 12 shows the outlets 44 and 45 on an enlarged scale. The device 1b shown in Figure 11 allows different forms of liquid to be supplied to the electrohydrodynamic processing site provided by the outlets 44 and 45.

The reservoir 20a coupled to the inner outlet 44 may contain a supply of a biologically active ingredient such as a pharmaceutical or a solution of DNA for example, while the reservoir 20b coupled to the outer nozzle 45 may contain a supply of a polymer solution of the type discussed above, for example polyhydroxybutyric acid dissolved in methylene chloride. The device shown in Figure 11 is operated in a similar manner to the device shown in Figure 3. Thus, the switch SW1 is first activated to supply the required voltages, typically 10 to 25kv, the flow regulating valves 50a and 50b are then opened to provide the required flow from each of the nozzles 44 and 45 and the pumps 100a and 100b and valves 11a and 11b, if present, activated to supply liquid to the respective nozzles 44 and 45. The outlets of the two coaxial nozzles are designed to promote laminar flow so that the polymer containing solution issues from the nozzle 45 so as to surround the other liquid.

By appropriate selection the molecular weight of the polymer and/or the volatility of the polymer solution, the liquids issuing from the combined nozzle can be caused to form a fibre or fibrils in which the biologically active ingredient forms a cylindrical centre core of the fibre or fibril or micro capsules in which the biologically active ingredient is completely encapsulated within the polymer and may still be in a liquid form.

For microcapsule formation it has been found preferable to reduce the percentage polymer in solution and to use a much reduced molecular weight polymer. For example a resin such as neoprene chlorinated rubber dissolved at more than about 10% by weight in trichloroethane will tend to spray fibres. However by decreasing the percentage polymer and/or using a less volatile solvent such as, for example, propylene glycol ether, microcapsules may be formed. Microcapsules have been produced using PVA of low molecular weight, for example a molecular weight of about 15000, dissolved to a dilution of between about 2.5 per cent and 5 per cent by volume in water or alcohol with a flow rate of about 1.0 microlitres per second. Production of microcapsules may be enhanced by using two reactive monomers one of which is placed in each of the two liquids to react during comminution.

Composite products embodying the invention produced using the device shown in Figures 11 and 12 may be used to form a dressing in the manner described above where the composite product is in the form of fibres or long fibrils allowing for controlled release of the active ingredient as the bioresorbable polymer degrades. Where the composite products produced are fibres, fibrils or microcapsules, then these may be applied to the surface of the skin or into a wound in combination with, for example, a conventional dressing or a dressing produced from comminuted fibres. Material from the core of a fibre or fibril may be released from the ends of the fibre or fibril. Material from the core of a fibre, fibril or microcapsule may be released through the coating if the coating is permeable to the material contained within it or may be released as a result of the outer coating being breached, for example by chemical or enzymic attack which causes the outer coating to dissolve or degrade, by bioresorption or biodegradation of the coating, or as a result of temperature changes or application of pressure which causes the outer coating to rupture.

Composite products made up of three or more different layers of material may be formed by increasing the number of coaxial nozzles.

The outlet nozzle of the device shown in Figure 11 may comprise a number of sets of coaxial outlet nozzles 44 and 45 in a manner similar to that shown in Figure 7 for single outlet nozzles. This would allow different active ingredients to be supplied to different ones of the inner nozzles 44. The different active ingredients can thus be kept apart until actual use which is of particular advantage where the active ingredients react to form a product which itself has a low shelf life.

It will, of course, be appreciated that the device shown in Figure 1 could be modified in a manner similar to that shown in Figure 11 for Figure 3 to produce a device embodying the invention capable of forming cored fibres, fibrils or microcapsules.

As discussed above, the nozzle shown in Figure 12 is deliberately designed to avoid mixing between the two liquids which are generally selected so as to be immiscible thereby enabling production of a cored fibre, fibril or microcapsule.

Figure 13 shows an alternative form of nozzle which may be used in the device shown in Figure 11 but does not fall within the scope of the invention claimed. The nozzle shown in Figure 13 is a slot-nozzle similar to that shown in Figure 8 but provided with two separate channels 46 and 47 coupled to respective ones of the liquid supply pipes so that each channel receives a different liquid. The outlets of the channels 46 and 47 are designed so as to create turbulence and therefore mixing of two liquids at the outlet. This arrangement may be used where, for example, it is desired to have some control over the amount of active ingredient which may be incorporated into a liquid or to combine two liquids which then react. A polyurethane foam has been formed by reacting a solution of urethane supplied via one of the nozzles with a blowing agent supplied by the other nozzle to spray a flexible foam deposit into a wound to form a cavity wound dressing. This arrangement has the advantage that the dressing will conform to the contours of a cavity wound and may be applied with clerical cleanliness without handling. Again, an active ingredient such as a pharmaceutically active ingredient may be incorporated into one of the two liquids or mixed with the two liquids.

The nozzle shown in Figure 13 may also be used to, for example, bring reactive liquids together at the nozzle to deposit reacting or reactive product onto the skin or into a wound which should be of advantage where the reactive product has a very short lifetime and cannot be stored. For example, the nozzle shown in Figure 13 has been used experimentally to produce a fibrin mat by supplying the enzyme thrombin to one channel and fibrinogen to the other channel.

As another possibility the device shown in Figure 11 may be modified to provide two separate spaced nozzles and the voltage source arranged to charge the two nozzles to voltages of opposite polarity in a manner similar to that described in WO94/12285 so as to enable liquid droplets charged to one polarity to rapidly coalesce with droplets charged to the other polarity to form ultra-small particles of from sub-micron to a few tens of microns in diameter. Again, for example, ultra small droplets containing, for example the enzyme thrombin may be sprayed at one polarity so as to rapidly coalesce with droplets of the opposite polarity containing fibrinogen to deposit a fast reacting fibrin mat to cause blood clotting, for wound sealing or for adhesion.

A method embodying the invention may also be used to produce material capable of transfecting resident cells in situ with genetic material in order to regulate cell responses. For example, a method embodying the invention may be used to produce microcapsules comprising DNA encapsulated in a microcapsules or complexed with an appropriate lipid material for transfecting cells. Phospholipid microcapsules encapsulating DNA may be produced by a method embodying the invention. Other biological material such as proteins may be similarly encapsulated or complexed with an appropriate lipid material. Proteins may also be incorporated in the lipid layer. Surface binding or surface recognising agents such as surface protein A may be incorporated into microcapsules, especially phospholipid microcapsules, for selecting targets such as cancer cells, epithelial cells etc. Also, surfactants such as soya lecithin available from Sigma Pharmaceuticals may be incorporated in the outer surface of fibres, microcapsules or fibrils.

Fibres, fibrils or droplets or capsules produced by a method embodying the invention may be coated with substances such as surfactants such as soya lecithin or with, for example, DNA which is relatively sticky. This may be achieved by, for example, supplying the polymer containing liquid to the inner nozzle in Figure 11 and supplying the coating material to the outer nozzle in Figure 11. Alternatively, a separate spraying device, which may be a conventional or electrohydrodynamic spraying device, may be provided so as to direct, for example, an oppositely charged spray or cloud of the coating material into the path of the material produced by the apparatus shown in Figure 1, 2 or 11, for example.

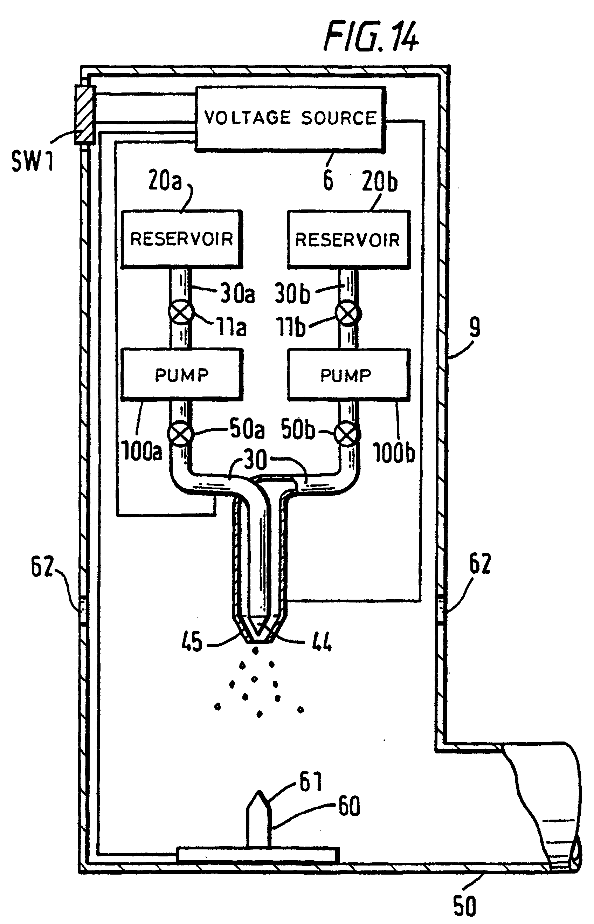

Figure 14 illustrates schematically a modified form of the device shown in Figure 11 which may be suitable for producing fibrils or microcapsules for inhalation. The device shown in Figure 14 differs from that shown in Figure 11 merely by the provision of air vents 62 and electrical discharge 60 means for discharging the fibrils or microcapsules and an outlet 50 adapted to receive a tube for insertion into the mouth or trachea of a user or to receive a mask to cover the mouth and nose of a user where both oral and nasal inhalation are required. The electrical discharging means may comprise, for example, an earthed discharge electrode 61 so as to produce gaseous ions of the opposite polarity to the charged fibrils or microcapsules so that the fibrils or microcapsules are discharged for inhalation by a user. The discharging means may be brought into operation by the active inhalation by the user as described in, for example, WO94/14543. The provision of the electrical discharge means enables the fibrils or microcapsules to be delivered to the upper or lower regions of the lungs rather than simply to the nasal passages. The actual location to which the fibrils or microcapsules are delivered can be controlled by controlling any residual electrical charge and the precise dimensions of the fibrils or microcapsules may be controlled by controlling the volatility, flow rate and voltage applied to the nozzle.

The material for oral delivery may comprise liposome encapsulated or complexed DNA for transfecting cells or may, for example comprise biologically active ingredients such as peptides, polypeptides and other large biomolecules such as insulin or growth factor, and active pharmaceutical components for enabling delivery of the active component into the blood stream via the lung. This should provide a quicker route to the bloodstream than that provided by normal oral ingestion and avoids the need for injection of components which cannot be taken orally because of the gastric enzymes and acids present in the digestive system.

In a method embodying the invention used to produce fibres, fibrils or microcapsules comprising a core of an active ingredient, the choice of coating material, the permeability and/or thickness of the coating may be adjusted to adjust the timing of release of the active ingredient. For example where the coating comprises a bioresorbable or biodegradable polymer, the half-life of the polymer may be controlled by controlling the permeability and/or thickness of the polymer coating by, for a specific formulation, controlling the flow rate and voltage.

A method embodying the invention may also be used to supply material to body cavities other than the respiratory system. Generally, for such use, the material will be at least partially electrically discharged before supply and means may be provided for forming an air or inert gas flow to assist the supply of the material to the body cavity. Where the body cavity is not easily accessible from the outside of the body, then the device embodying the invention may be mounted to an endoscope or like instrument enabling the device to be inserted into the body and to be positioned at the site where the material is required. The material may comprise any of the fibres, fibrils, particles and microcapsules mentioned above.

A method embodying the invention may also be used in a production process to form fibrils or particles comprising a biologically active ingredient and/or fibrils or microcapsules having a core of a biologically active ingredient which may themselves be encapsulated in conventional orally ingestible capsules, enabling, especially in the case of microcapsules, good control over the release of the active material.

A method and device embodying the invention may also be used for non-medical purposes. For example, coatings of fibres, fibrils, particles or microcapsules may be formed on substrates such as paper with good control of the thickness and uniformity of the coating. For example, adhesive may be deposited onto a substrate using a method embodying the invention.

Materials formed of two or more components which have only a short-shelf life when mixed together may be formed in a timely manner using a method embodying the present invention by encapsulating the respective components in respective fibres, fibrils or microcapsules so that mixing of the various components only occurs when the components are released from the encapsulating material by, for example, leaching through the encapsulating material, rupture by pressure being applied to the encapsulating material, temperature, or degradation, for example bioresorption or biodegradation, of the encapsulant. Such a method may be used to form, for example, two component adhesives which may be applied separately or simultaneously to a surface as cored fibres, fibrils or microcapsules by a method embodying the invention.

Other materials such as perfumes, insecticides, aromas, vapours, inks, dyes, lubricants, insect repellents etc., may be encapsulated in fibres, fibrils or microcapsules and deposited on a surface using a method embodying the invention, allowing the encapsulated ingredient to be released in a time-controlled manner as discussed in the previous paragraph, for example by application of pressure to the surface.

A method embodying the invention may also be used to produce a protective coating which may contain an active protective ingredient such as an anti-corrosive or a lubricant. For example, temporary protective coatings of delicate articles or articles liable to corrosion may be provided by depositing a web or mat on the surface of the article using a method embodying the invention.

webs or mats formed using a method embodying the invention may also be sprayed or deposited over, for example, delicate crops such as grapes or strawberries so as to protect them from environmental effects such as frost, sun-damage, etc. Such a web may incorporate active ingredients such as insecticides, fungicides, miticides and the like to further protect the crop.

Generally, the capacitive nature of materials such as skin and the moisture content of the air should be sufficient for deposition onto a surface to occur simply by electrostatic attraction. However, where it is desired to deposit a large amount of material, then it may be necessary to earth the surface or to maintain it at a lower or opposite potential to the charged matter.

A method and device embodying the invention may also be used for supplying material to cavities other than body cavities and to concave surfaces. In such circumstances, the charged matter will generally be at least partially electrically discharged before it reaches the cavity or concave surface.

As used herein, the term "particle" includes solid, partially solid and gel-like droplets and microcapsules which incorporate solid, partially solid, gel-like or liquid matter. The term "active ingredient" means material which is compatible with and has an affect on the substrate or body to which it is to be applied and the term "biologically active ingredient" or "biologically active material" means material which is compatible with and has an affect (which may for example be biological, chemical or biochemical) on the animal or plant to which it is to be applied and includes, for example, medicaments such as proprietary medicines, pharmaceutical medicines and veterinary medicines, vaccines, genetic material such as DNA, cells and the like.

## Claims

1. A method of forming composite matter, which comprises supplying a liquid to a first outlet (44) and supplying a further liquid to a second outlet (45) located adjacent the first outlet and subjecting the liquids to an electric field so as to form at least one jet, the liquids being such that, after formation, the jet forms composite matter comprising at least one of a fibre, fibre fragments and particles, the composite matter having a core formed substantially by one of the two liquids and a coating formed substantially by the other of the two liquids.

2. A method according to claim 1, which further comprises positioning the first and second outlets in the vicinity of a substrate such that the composite matter deposits onto the substrate to form a mat or web on the substrate.

3. A method according to claim 2, which comprises repeating or continuing the deposition process to deposit a number of layers of composite matter one on top of the other.

4. A method according to claim 2 or 3, which further comprises depositing a different type of material onto said substrate.

5. A method according to claim 4, which comprises depositing the different material by electrohydrodynamically processing a different liquid to form material comprising at least one of a fibre, fibre fragments and particles.

6. A method according to any one of claims 2 to 5, which comprises effecting relative movement between the at least one jet and the substrate during deposition.

7. A method according to any one of the preceding claims, wherein the composite matter is electrically charged and the method further comprises at least partially electrically discharging the composite matter.

8. A method according to any one of claims 2 to 6, wherein the composite matter is electrically charged and the substrate is a cavity or concave surface.

9. A method according to any one of claims 2 to 6, wherein the composite matter is electrically charged and the substrate is a cavity or concave surface and the method further comprises at least partially electrically discharging the comminuted matter prior to supply to the cavity or concave surface.

10. A method according to claim 9, which further comprises supplying the at least partially electrically discharged matter to the said cavity or concave surface from a location remote from said cavity or concave surface.

11. A method according to any one of the preceding claims, wherein at least one of the liquids comprises a biologically active ingredient.

12. A method according to any one of claims 1 to 10, wherein at least one of the liquids comprises one or more biologically active ingredients selected from the group consisting of a proteolytic enzyme, a cytokine, a growth factor such as one of fibroblast growth factor, epithelial growth factor and transforming growth factor, collagen, fibrinogen, an antibiotic, an antiseptic, an antifungal, an analgesic, an antiparasitic, a batericide, DNA or other genetic matter, cells, a peptide or polypeptide, insulin, an adjuvant, an immune suppressant or stimulant, a surface binding or surface recognising agent such as surface protein A, a surfactant, and a vaccine.

13. A method according to claim 11, wherein at least one of the liquids comprises one or more biologically active ingredients selected from the group consisting of DNA, a peptide or polypeptide, insulin, and growth factor.

14. A method according to any one of the preceding claims, wherein one of the liquids comprises a polymer or resin.

15. A method according to any one of the preceding claims, wherein one of the liquids comprises a bioresorbable or biodegradable material.

16. A method according to any one of claims 1 to 13, wherein at least one of the liquids comprises polyvinyl alcohol, polyhydroxybutyric acid, polyglycolic acid, polylactic acid, nitrocellulose or a polysaccharide.

17. A method according to any one of the preceding claims, which comprises coating a fibre, fibre fragment or particle with another material.

18. A method according to claim 16, which comprises coating a fibre, fibre fragment or particle with a biologically active material such as DNA, a surfactant, a surface recognition protein or a lipid.

19. A method according to any one of claims 1 to 13, wherein the liquid forming the coating comprises a bioresorbable or biodegradable polymer whereby, when the composite matter is ingested, the polymer is degraded by chemical or enzymic attack to enable release of an active ingredient from the core.

20. A method according to any one of claims 2 to 10, further comprising incorporating cells into the deposited mat or web.

21. A method according to claim 3, which further comprises interspersing skin cells in or between said layers.

22. A method according to claim 2 or 3, further comprising incorporating cytokines into the mat or web.

23. A device comprising a housing (9) containing:
a first reservoir (20a) containing a first liquid;
a second reservoir (20b) containing a second liquid;
first liquid supplying means for supplying the first liquid to a first liquid supply outlet (44);
second supplying means for supplying the second liquid to a second liquid outlet (45) adjacent to the first liquid outlet; and
means (6) for subjecting liquid issuing from the first and second liquid outlets to an electric field to cause the liquids to form at least one jet of electrically charged liquid, the liquids being such that, in use of the device after the at least one jet has been formed, the jet forms composite matter comprising at least one of a fibre, fibre fragments and particles, the composite matter having a core formed substantially by one of the first and second liquids and a coating formed substantially by the other of the first and second liquids.

24. A device according to claim 23, wherein the housing (9) also contains discharging means for at least partially electrically discharging charged composite matter.

25. A device according to claim 23 or 24, further comprising means for providing an air or inert gas flow to assist supply of the composite matter from the housing outlet.

26. A device according to claim 23, 24 or 25, wherein the housing is arranged to be held by a hand.

27. A device according to any one of claims 23, 24 or 25, wherein at least one of the first and second liquids comprises a biologically active ingredient.

28. A device according to any one of claims 23 to 25, wherein at least one of the first and second liquids comprises one or more biologically active ingredients selected from the group consisting of a proteolytic enzyme, a cytokine, a growth factor such as one of fibroblast growth factor, epithelial growth factor and transforming growth factor, collagen, fibrinogen, an antibiotic, an antiseptic, an antifungal, an analgesic, an antiparasitic, a batericide, DNA or other genetic matter, cells, a peptide or polypeptide, insulin, an adjuvant, an immune suppressant or stimulant, a surface binding or surface recognising agent such as surface protein A, a surfactant, and a vaccine.

29. A device according to claim 27, wherein at least one of the first and second liquids comprises one or more biologically active ingredients selected from the group consisting of DNA, a peptide or polypeptide, insulin, and growth factor.

30. A device according to any one of claims 23 to 29, wherein one of the first and second liquids comprises a polymer or resin.

31. A device according to any one of claims 23 to 29, wherein one of the first and second liquids comprises a bioresorbable or biodegradable material.

32. A device according to any one of claims 23 to 29, wherein one of the first and second liquids comprises animal collagen or fibrinogen.

33. A device according to any one of claims 23 to 29, wherein the or at least one of the first and second liquids comprises polyvinyl alcohol, polyhydroxybutyric acid, polyglycolic acid, polylactic acid, nitrocellulose or a polysaccharide.

34. An inhaler comprising a device in accordance with claim 23.

## Patentansprüche

1. Verfahren zum Bilden eines Verbundstoffs, in dem eine Flüssigkeit einem ersten Auslaß (44) zugeführt wird, eine weitere Flüssigkeit einem zweiten neben dem ersten Auslaß angeordneten Auslaß (45) zugeführt wird und die Flüssigkeiten einem elektrischen Feld ausgesetzt werden, so daß wenigstens ein Strahl ausgebildet wird, wobei die Flüssigkeiten so beschaffen sind, daß der Strahl nach seiner Ausbildung einen Verbundstoff bildet, der eine Faser, Faserfragmente und/oder Teilchen enthält, und der Verbundstoff einen im wesentlichen durch eine der beiden Flüssigkeiten gebildeten Kern, sowie einen im wesentlichen durch die andere der beiden Flüssigkeiten gebildeten Überzug aufweist.

2. Verfahren nach Anspruch 1, wobei ferner der erste und der zweite Auslaß in der Nähe eines Substrats angeordnet wird, so daß sich der Verbundstoff auf dem Substrat ablagert, um eine Matte oder ein Netz auf diesem zu bilden.

3. Verfahren nach Anspruch 2, wobei der Ablagerungsprozeß wiederholt oder fortgesetzt wird, um mehrere Schichten Verbundstoff übereinander abzulagern.

4. Verfahren nach Anspruch 2 oder 3, wobei ferner ein Material unterschiedlichen Typs auf dem Substrat abgelagert wird.

5. Verfahren nach Anspruch 4, wobei das unterschiedliche Material durch elektrohydrodynamische Verarbeitung einer unterschiedlichen Flüssigkeit abgelagert wird, um ein Material zu bilden, das eine Faser, Faserfragmente und/oder Teilchen enthält.

6. Verfahren nach einem der Ansprüche 2 bis 5, wobei eine Relativbewegung zwischen dem wenigstens einen Strahl und dem Substrat während des Ablagerns bewirkt wird.

7. Verfahren nach einem der vorstehenden Ansprüche, wobei der Verbundstoff elektrisch geladen ist und in dem Verfahren ferner der Verbundstoff wenigstens teilweise elektrisch entladen wird.

8. Verfahren nach einem der Ansprüche 2 bis 6, wobei der Verbundstoff elektrisch geladen ist und das Substrat einen Hohlraum oder eine konkave Fläche darstellt.

9. Verfahren nach einem der Ansprüche 2 bis 6, wobei der Verbundstoff elektrisch geladen ist, das Substrat einen Hohlraum oder eine konkave Fläche darstellt und in dem Verfahren ferner der zerkleinerte Stoff wenigstens teilweise elektrisch entladen wird, bevor er dem Hohlraum oder der konkaven Fläche zugeführt wird.

10. Verfahren nach Anspruch 9, wobei ferner der wenigstens teilweise elektrisch entladene Stoff dem Hohlraum oder der konkaven Fläche von einer Stelle zugeführt wird, die von dem Hohlraum oder der konkaven Fläche entfernt ist.

11. Verfahren nach einem der vorstehenden Ansprüche, wobei wenigstens eine der Flüssigkeiten einen biologisch aktiven Inhaltsstoff aufweist.

12. Verfahren nach einem der Ansprüche 1 bis 10, wobei wenigstens eine der Flüssigkeiten mindestens einen der folgenden biologisch aktiven Inhaltsstoffe enthält: ein proteolytisches Enzym, ein Cytokin, einen Wachstumsfaktor, wie z.B. einen Fibroblasten-Wachstumsfaktor, einen Epithel-Wachstumsfaktor oder einen transformierenden Wachstumsfaktor, Collagen, Fibrinogen, ein Antibiotikum, ein Antiseptikum, ein Fungizid, ein Analgetikum, ein Antiparasitikum, ein Bakterizid, DNA oder einen anderen genetischen Stoff, Zellen, ein Peptid oder Polypeptid, Insulin, einen Hilfsstoff, ein Immunsuppresivum oder Stimulans, ein Oberflächenbindungs- oder Oberflächenerkennungsmittel, wie z.B. ein Oberflächenprotein A, einen oberflächenaktiven Stoff und/oder einen Impfstoff.

13. Verfahren nach Anspruch 11, wobei wenigstens eine der Flüssigkeiten mindestens einen der folgenden biologisch aktiven Inhaltsstoffe enthält: DNA, ein Peptid oder Polypeptid, Insulin und/oder einen Wachstumsfaktor.

14. Verfahren nach einem der vorstehenden Ansprüche, wobei eine der Flüssigkeiten ein Polymer oder Harz enthält.

15. Verfahren nach einem der vorstehenden Ansprüche, wobei eine der Flüssigkeiten ein biologisch resorbierbares oder biologisch abbaubares Material enthält.

16. Verfahren nach einem der Ansprüche 1 bis 13, wobei wenigstens eine der Flüssigkeiten Polyvinylalkohol, Polyhdydroxybuttersäure, Polyglycolsäure, Polymilchsäure, Nitrocellulose oder Polysaccharid enthält.

17. Verfahren nach einem der vorstehenden Ansprüche, wobei eine Faser, ein Faserfragment oder Teilchen mit einem anderen Material überzogen wird.

18. Verfahren nach Anspruch 16, wobei eine Faser, ein Faserfragment oder Teilchen mit einem biologisch aktiven Material, wie z.B. DNA, einem oberflächenaktiven Wirkstoff, einem Oberflächenerkennungsprotein oder einem Lipid überzogen wird.

19. Verfahren nach einem der Ansprüche 1 bis 13, wobei die den Überzug bildende Flüssigkeit ein biologisch resorbierbares oder biologisch abbaubares Polymer enthält, wodurch, wenn der Verbundstoff eingenommen wird, das Polymer durch einen Chemie- oder einen Enzymangriff abgebaut wird und so die Freigabe eines aktiven Inhaltsstoffs aus dem Kern ermöglicht.

20. verfahren nach einem der Ansprüche 2 bis 10, wobei ferner Zellen in die abgelagerte Matte oder das abgelagerte Netz eingebaut werden.

21. Verfahren nach Anspruch 3, wobei ferner Hautzellen in oder zwischen den Schichten eingeschoben werden.

22. Verfahren nach Anspruch 2 oder 3, wobei ferner Cytokine in die Matte oder das Netz eingebaut werden.

23. Vorrichtung mit einem Gehäuse (9), umfassend:
ein erstes Reservoir (20a), das eine erste Flüssigkeit enthält;
ein zweites Reservoir (20b), das eine zweite Flüssigkeit enthält;
eine erste Flüssigkeitszuführeinrichtung, um die erste Flüssigkeit einem ersten Flüssigkeitszuführauslaß (44) zuzuführen;
eine zweite Zuführeinrichtung, um die zweite Flüssigkeit einem zweiten Flüssigkeitsauslaß (45) zuzuführen, der neben dem ersten Flüssigkeitsauslaß angeordnet ist; und
eine Einrichtung (6), um aus dem ersten und dem zweiten Flüssigkeitsauslaß austretende Flüssigkeit einem elektrischen Feld auszusetzen, damit die Flüssigkeiten wenigstens einen Strahl elektrisch geladener Flüssigkeit ausbilden, wobei die Flüssigkeiten so beschaffen sind, daß der Strahl bei Benutzung der Vorrichtung, nachdem wenigstens ein Strahl ausgebildet wurde, einen Verbundstoff bildet, der eine Faser, Faserfragmente und/oder Teilchen enthält, und der verbundstoff einen im wesentlichen durch eine der beiden Flüssigkeiten gebildeten Kern, sowie einen im wesentlichen durch die andere der beiden Flüssigkeiten gebildeten Überzug aufweist.

24. Vorrichtung nach Anspruch 23, wobei das Gehäuse (9) ferner eine Entladungseinrichtung enthält, um den geladenen Verbundstoff wenigstens teilweise elektrisch zu entladen.

25. Vorrichtung nach Anspruch 23 oder 24, ferner mit einer Einrichtung zum Liefern einer Luft- oder Inertgas-Strömung, um das Zuführen des Verbundstoffs aus dem Gehäuseauslaß zu unterstützen.

26. Vorrichtung nach Anspruch 23, 24 oder 25, wobei das Gehäuse dazu ausgelegt ist, in der Hand gehalten zu werden.

27. Vorrichtung nach einem der Ansprüche 23, 24 oder 25, wobei wenigstens eine der beiden Flüssigkeiten einen biologisch aktiven Inhaltsstoff enthält.

28. Vorrichtung nach einem der Ansprüche 23 bis 25, wobei wenigstens eine der beiden Flüssigkeiten mindestens einen der folgenden biologisch aktiven Inhaltsstoffe enthält: ein proteolytisches Enzym, ein Cytokin, einen Wachstumsfaktor, wie z.B. einen Fibroblasten-Wachstumsfaktor, einen Epithel-Wachstumsfaktor oder einen transformierenden Wachstumsfaktor, Collagen, Fibrinogen, ein Antibiotikum, ein Antiseptikum, ein Fungizid, ein Analgetikum, ein Antiparasitikum, ein Bakterizid, DNA oder einen anderen genetischen Stoff, Zellen, ein Peptid oder Polypeptid, Insulin, einen Hilfsstoff, ein Immunsuppresivum oder Stimulans, ein Oberflächenbindungs- oder Oberflächenerkennungsmittel, wie z.B. ein Oberflächenprotein A, einen oberflächenaktiven Stoff und/oder einen Impfstoff.

29. Vorrichtung nach Anspruch 27, wobei wenigstens eine der beiden Flüssigkeiten mindestens einen der folgenden biologisch aktiven Inhaltsstoffe enthält: DNA, ein Peptid oder Polypeptid, Insulin und/oder einen Wachstumsfaktor.

30. Vorrichtung nach einem der Ansprüche 23 bis 29, wobei eine der beiden Flüssigkeiten ein Polymer oder Harz enthält.

31. Vorrichtung nach einem der Ansprüche 23 bis 29, wobei eine der beiden Flüssigkeiten ein biologisch resorbierbares oder biologisch abbaubares Material enthält.

32. Vorrichtung nach einem der Ansprüche 23 bis 29, wobei die erste oder die zweite Flüssigkeit ein Tiercollagen oder Fibrinogen enthält.

33. Vorrichtung nach einem der Ansprüche 23 bis 29, wobei wenigstens eine der beiden Flüssigkeiten Polyvinylalkohol, Polyhdydroxybuttersäure, Polyglycolsäure, Polymilchsäure, Nitrocellulose oder Polysaccharid enthält.

34. Inhaliergerät mit einer Vorrichtung nach Anspruch 23.

## Revendications

1. Procédé de formation d'une substance composite, comprenant la fourniture d'un liquide à une première sortie (44) et la fourniture d'un liquide supplémentaire à une deuxième sortie (45) située en position contiguë à la première sortie et l'exposition des liquides à un champ électrique de manière à former au moins un jet, les liquides étant tels que, après formation, le jet forme une substance composite comprenant au moins l'un parmi une fibre, des fragments de fibre et des particules, la substance composite ayant un noyau formé sensiblement d'un des deux liquides et un revêtement formé sensiblement par l'autre des deux liquides.

2. Procédé selon la revendication 1, comprenant en outre le positionnement des première et deuxième sorties à proximité d'un substrat de telle manière que la substance composite se dépose sur le substrat pour former un mat ou une toile sur le substrat.

3. Procédé selon la revendication 2, comprenant la répétition ou la continuation du processus de dépôt pour déposer plusieurs couches de substance composite l'une au-dessus de l'autre.

4. Procédé selon la revendication 2 ou 3, comprenant en outre le dépôt d'un type de matériau différent sur ledit substrat.

5. Procédé selon la revendication 4, comprenant le dépôt du matériau différent par traitement électro-hydrodynamique d'un liquide différent pour former un matériau comprenant au moins l'un parmi une fibre, des fragments de fibre et des particules.

6. Procédé selon l'une quelconque des revendications 2 à 5, comprenant la réalisation d'un mouvement relatif entre au moins un jet et le substrat lors du dépôt.

7. Procédé selon l'une quelconque des revendications précédentes, dans lequel la substance composite est électriquement chargée et le procédé comprend en outre plus la décharge électrique au moins partielle de la substance composite.

8. Procédé selon l'une quelconque des revendications 2 à 6, dans lequel la substance composite est électriquement chargée et le substrat est une cavité ou une surface concave.

9. Procédé selon l'une quelconque des revendications 2 à 6, dans lequel la substance composite est électriquement chargée et le substrat est une cavité ou une surface concave et le procédé comprend en outre la décharge électrique au moins partielle de substance broyée avant de l'appliquer sur la cavité ou surface concave.

10. Procédé selon la revendication 9, comprenant en outre l'application de la substance au moins partiellement déchargée sur ladite cavité ou surface concave depuis un emplacement distant de ladite cavité ou surface concave.

11. Procédé selon l'une quelconque des revendications précédentes, dans lequel au moins un des liquides comprend un ingrédient biologiquement actif.

12. Procédé selon l'une quelconque des revendications 1 à 10, dans lequel au moins un des liquides comprend un ou plusieurs ingrédients biologiquement actifs choisis dans le groupe consistant en une enzyme protéolytique, une cytokine, un facteur de croissance tel que l'un parmi le facteur de croissance de fibroblastes, le facteur de croissance épithélial et le facteur de croissance transformant, le collagène, le fibrinogène, un antibiotique, un antiseptique, un antifongique, un analgésique, un antiparasitaire, un bactéricide, de l'ADN ou un autre matériau génétique, des cellules, un peptide ou un polypeptide, l'insuline, un adjuvant, un suppresseur ou stimulant immunitaire, un agent de liaison de surface ou de reconnaissance de surface tel que la protéine de surface A, un surfactant, et un vaccin.

13. Procédé selon la revendication 11, dans lequel au moins un des liquides comprend un ou plusieurs ingrédients biologiquement actifs choisis dans le groupe consistant en l'ADN, un peptide ou un polypeptide, l'insuline, et un facteur de croissance.

14. Procédé selon l'une quelconque des revendications précédentes, dans lequel un des liquides comprend un polymère ou une résine.

15. Procédé selon l'une quelconque des revendications précédentes, dans lequel un des liquides comprend un matériau biorésorbable ou biodégradable.

16. Procédé selon l'une quelconque des revendications 1 à 13, dans lequel au moins un des liquides comprend de l'alcool polyvinylique, de l'acide polyhydroxybutyrique, de l'acide polyglycolique, de l'acide polylactique, de la nitrocellulose ou un polysaccharide.

17. Procédé selon l'une quelconque des revendications précédentes, comprenant le revêtement d'une fibre, d'un fragment de fibre ou d'une particule avec un autre matériau.

18. Procédé selon la revendication 16, comprenant le revêtement d'une fibre, d'un fragment de fibre ou d'une particule avec un matériau biologiquement actif tel que l'ADN, un surfactant, une protéine de reconnaissance de surface ou un lipide.

19. Procédé selon l'une quelconque des revendications 1 à 13, dans lequel le liquide formant le revêtement comprend un polymère biorésorbable ou biodégradable de manière à ce que, lorsque la substance c omposite est ingérée, le polymère soit dégradé par attaque chimique ou enzymatique pour permettre la libération d'un ingrédient actif depuis le noyau.

20. Procédé selon l'une quelconque des revendications 2 à 10, comprenant en outre l'incorporation de cellules dans le mat ou la toile déposé.

21. Procédé selon la revendication 3, comprenant en outre l'intercalation de cellules de peau dans ou entre lesdites couches.

22. Procédé selon la revendication 2 ou 3, comprenant en outre l'incorporation de cytokines dans le mat ou la toile.

23. Dispositif comprenant un boîtier (9) contenant :
un premier réservoir (20a) contenant un premier liquide ;
un deuxième réservoir (20b) contenant un deuxième liquide ;
un premier moyen de fourniture de liquide pour fournir le premier liquide à une première sortie de fourniture de liquide (44) ;
un deuxième moyen de fourniture de liquide pour fournir le deuxième liquide à une deuxième sortie de fourniture de liquide (45) contiguë à la première sortie de liquide ; et
un moyen (6) pour exposer le liquide sortant des première et deuxième sorties de liquide à un champ électrique pour amener les liquides à former au moins un jet de liquide électriquement chargé, les liquides étant tels que, dans l'utilisation du dispositif après qu'au moins un jet ait été formé, le jet forme une substance composite comprenant au moins l'un parmi une fibre, des fragments de fibre et des particules, la substance composite ayant un noyau formé sensiblement par un des premier et deuxième liquides et un revêtement formé sensiblement par l'autre des premier et deuxième liquides.

24. Dispositif selon la revendication 23, dans lequel le boîtier (9) contient également un moyen de décharge pour décharger électriquement, au moins partiellement, la substance composite.

25. Dispositif selon la revendication 23 ou 24, comprenant en outre un moyen pour fournir un courant d'air ou de gaz inerte pour faciliter la fourniture de la substance composite depuis la sortie du boîtier.

26. Dispositif selon la revendication 23, 24 ou 25, dans lequel le boîtier agencé pour être tenu par une main.

27. Dispositif selon l'une quelconque des revendications 23, 24 ou 25, dans lequel au moins un des premier et deuxième liquides comprend un ingrédient biologiquement actif.

28. Dispositif selon l'une quelconque des revendications 23 à 25, dans lequel au moins un des premier et deuxième liquides comprend un ou plusieurs ingrédients biologiquement actifs choisis dans le groupe consistant en une enzyme protéolytique, une cytokine, un facteur de croissance tel que l'un parmi le facteur de croissance de fibroblastes, le facteur de croissance épithélial et le facteur de croissance transformant, le collagène, le fibrinogène, un antibiotique, un antiseptique, un antifongique, un analgésique, un antiparasitaire, un bactéricide, de l'ADN ou un autre matériau génétique, des cellules, un peptide ou un polypeptide, l'insuline, un adjuvant, un suppresseur ou stimulant immunitaire, un agent de liaison de surface ou de reconnaissance de surface tel que la protéine de surface A, un surfactant, et un vaccin.

29. Dispositif selon la revendication 27, dans lequel au moins un des premier et deuxième liquides comprend un ou plusieurs ingrédients biologiquement actifs choisis dans le groupe consistant en l'ADN, un peptide ou un polypeptide, l'insuline, et un facteur de croissance.

30. Dispositif selon l'une quelconque des revendications 23 à 29, dans lequel un des premier et deuxième liquides comprend un polymère ou une résine.

31. Dispositif selon l'une quelconque des revendications 23 à 29, dans lequel un des premier et deuxième liquides comprend un matériau biorésorbable ou biodégradable.

32. Dispositif selon l'une quelconque des revendications 23 à 29, dans lequel un des premier et deuxième liquides comprend un collagène ou fibrinogène animal.

33. Dispositif selon l'une quelconque des revendications 23 à 29, dans lequel l'au moins un des premier et deuxième liquides comprend de l'alcool polyvinylique, de l'acide polyhydroxybutyrique, de l'acide polyglycolique, de l'acide polylactique, de la nitrocellulose ou un polysaccharide.

34. Inhalateur comprenant un dispositif selon la revendication 23.
